# EUROPÄISCHE PATENTSCHRIFT

(11) **EP 3 424 446 B1**
(45) Veröffentlichungstag und Bekanntmachung des Hinweises auf die Patenterteilung: **29.01.2020**
(21) Anmeldenummer: 17180062.6
(22) Anmeldetag: 06.07.2017
(51) Int. Cl.: A61B 17/29, A61B 18/14

(54) **CHIRURGISCHES INSTRUMENT MIT ZWEISTUFIGEM BETÄTIGUNGSGETRIEBE**
SURGICAL INSTRUMENT WITH TWO STAGE ACTUATING MECHANISM
INSTRUMENT CHIRURGICAL DOTÉ D'UN MÉCANISME D'ACTIONNEMENT À DEUX NIVEAUX

(43) Veröffentlichungstag der Anmeldung: 09.01.2019
(73) Patentinhaber: Erbe Elektromedizin GmbH, 72072 Tübingen (DE)
(72) Erfinder: Kirstgen, Udo, 72108 Rottenburg (DE); Buntrock, Volker, 72762 Reutlingen (DE)
(74) Vertreter: Rüger Abel Patentanwälte PartGmbB

(56) Entgegenhaltungen:
- EP-A2- 2 000 100
- EP-B1- 2 845 549
- DE-A1-102010 025 550
- US-A- 5 817 093
- US-A1- 2013 053 831

## Beschreibung

Die Erfindung betrifft ein chirurgisches Instrument zur Durchführung chirurgischer Eingriffe an einem Patienten. Insbesondere handelt es sich dabei um ein Instrument, dessen Werkzeug Werkzeugteile aufweist, die zeitlich versetzt zu betätigen, d.h. zu bewegen sind, um einen gewünschten chirurgischen Effekt zu erzielen.

Ein solches Instrument ist prinzipiell aus der EP 2 845 549 B1 bekannt, aus dem die Merkmale des Oberbegriffs des Patentanspruchs 1 hervorgehen. Es weist ein an einem länglichen Schaft gehaltenes Werkzeug auf, zu dem ein Zangenwerkzeug und ein Schneidwerkzeug gehören. Das Zangenwerkzeug umfasst zwei Branchen, von denen wenigstens eine beweglich gelagert ist, so dass zwischen den beiden Branchen Gewebe, beispielsweise ein Blutgefäß oder dergleichen, gefasst und zusammengedrückt werden kann. Die Branchen tragen Elektroden zum Koagulieren von Gewebe und Fusionieren von Gefäßen. Das Schneidwerkzeug wird hingegen durch ein linear bewegliches an seiner Stirnseite eine Schneidkante aufweisendes Messer gebildet, das bei geschlossenen Branchen schneidend durch das gefasste und zusammengepresste Gefäß geschoben werden kann, um es zu durchtrennen.

Zur Betätigung der beiden Werkzeugteile ist an dem proximalen Ende des Schafts ein Gehäuse mit einem Handhebel vorgesehen. Das Gehäuse beherbergt ein Betätigungsgetriebe, das die Bewegung des Handhebels in eine Zugbewegung zum Schließen des Zangenwerkzeugs und in eine danach erfolgende Schubbewegung zum distalen Bewegen des Schneidwerkzeugs umsetzt. Das Betätigungsgetriebe enthält ein Kulissengetriebe zum Schließen des Zangenwerkzeugs. Das Kulissengetriebe weist eine Kulisse auf, die gelenkig mit dem Handhebel verbunden ist. Wird dieser geschwenkt, schwenkt auch die Kulisse des Kulissengetriebes, wodurch ein Kulissenstein in proximaler Richtung bewegt wird, um über ein Zugmittel das Zangenwerkzeug zu schließen. Die Betätigung des Schneidwerkzeugs erfolgt über einen mit einer Zahnstange versehenen Schlitten, der mit einem an dem Handhebel drehbar gehaltenen Zahnrad kämmt. An der gegenüber liegenden Seite des Zahnrads kämmt dieses mit einer in dem Gehäuse angebrachten ortsfesten Verzahnung. Solange das Kulissengetriebe aktiv ist, ist die Bewegung des zur Messerbetätigung vorgesehenen Schlittens gesperrt. Sobald die Kulisse ihre Bewegung beendet hat, wird die Bewegung des Schlittens in distaler Richtung freigegeben. Die Bewegung des Schlittens wird durch die von dem Handhebel verursachte Linearbewegung des an seinem Ende gelagerten Zahnrads bewirkt. Die Verhinderung der Messerbetätigung, d.h. der Linearbewegung des Zahnrads für den ersten Teil der Bewegung des Handhebels, erfordert eine Sperreinrichtung, deren Position und Gestaltung sorgfältig auf alle anderen Komponenten abgestimmt sein muss. Außerdem ergeben sich ein relativ großer Weg des Handhebels für das Schließen des Zangenwerkzeugs und ein relativ geringer Bedienweg für die Betätigung des Schneidwerkzeugs.

Es wird deswegen nach einem Konzept gesucht, mit dem sich die Bedienwege für die beiden beweglichen Werkzeugteile leicht aufeinander abstimmen lassen.

Diese Aufgabe wird mit dem Instrument nach Anspruch 1 gelöst.

Das erfindungsgemäße Instrument kann als laparoskopisches Instrument ausgebildet sein und dazu einen relativ steifen, dünnen und langen Schaft aufweisen, der an seinem distalen Ende ein Werkzeug trägt. Prinzipiell kann das Instrument mit den erfindungsgemäßen Merkmalen auch als Instrument für den offenchirurgischen Einsatz oder als endoskopisch einsetzbares Instrument gestaltet sein.

Das Instrument weist unabhängig davon ein Betätigungsgetriebe zur Bewegung der beiden Werkzeugteile auf, wobei das Betätigungsgetriebe sowohl ein Kurvengetriebe als auch ein Zahnsegmentgetriebe aufweist. Vorzugsweise ist das Kurvengetriebe darauf eingerichtet, eine proximal gerichtete Antriebsbewegung zu erzeugen, während das Zahnsegmentgetriebe vorzugsweise zur Erzeugung einer distal gerichteten Antriebsbewegung dient. Die Kurvenscheibe weist eine Außenumfangsfläche auf, an der ein Kurvenfolger, z.B. eine Rolle, ein Gleitkörper, ein Hebel oder dergleichen, anliegt. Der Kurvenfolger ist vorzugsweise in Radialrichtung zu der Kurvenscheibe bewegbar, wodurch seine Bewegungsrichtung die Drehachse der Kurvenscheibe schneidet. Die radiale Bewegungsrichtung des Kurvenfolgers stimmt vorzugsweise mit der Bewegungsrichtung des Zugmittels zum Antrieb des ersten Werkzeugteils überein. Zur Bewirkung einer Bewegung des Kurvenfolgers bei Drehung der Kurvenscheibe, weist diese einen Abschnitt auf, in dem der Radius der Außenumfangsfläche abhängig von dem Drehwinkel der Kurvenscheibe zu- oder abnimmt. Die Drehachse der Kurvenscheibe liegt dazu vorzugsweise auf der Mittelachse des Schafts. Dadurch wird von dem Kurvenfolger kein Drehmoment auf die Kurvenscheibe ausgeübt, sobald dieser den Kurvenscheibenabschnitt mit konstantem Radius erreicht hat.

Die beiden entgegengesetzt gerichteten, an den beiden Abtrieben des Betätigungsgetriebes abgreifbaren Antriebsbewegungen erfolgen vorzugsweise zeitlich versetzt, d.h. zeitlich überlappend oder nicht überlappend, jedenfalls nacheinander, wenn das Betätigungsgetriebe bewegt wird. Dazu dient vorzugsweise eine Handbetätigungseinrichtung, die dazu eingerichtet ist, eine Drehung sowohl einer zu dem Kurvengetriebe gehörigen Kurvenscheibe wie auch eine vorzugsweise gleichzeitige Drehung eines zu dem Zahnsegmentgetriebe gehörigen Segmentzahnrads zu bewirken. Die Handbetätigungseinrichtung kann z.B. ein an dem Gehäuse schwenkbar gelagerter Handhebel sein.

Das Zangenwerkzeug umfasst mindestens ein bewegliches Werkzeugteil, wie beispielsweise eine bewegliche Branche. Es können auch beide Branchen des Zangenwerkzeugs beweglich, z.B. aufeinander zu und voneinander weg schwenkbar sein. Die Bewegung des einen oder der beiden beweglichen Werkzeugteile des Zangenwerkzeugs wird von dem Betätigungsgetriebe ausgehend vorzugsweise über ein Zugmittel auf den oder die beweglichen Werkzeugteile des Zangenwerkzeugs übertragen. Das Zugmittel kann ein Kunststoff- oder Metalldraht, ein Kunststoff- oder Metallband, ein Kunststoff- oder Metallseil oder dergleichen sein und erstreckt sich vorzugsweise von dem proximalen Ende des Schafts, d.h. von dem ersten Abtrieb des Betätigungsgetriebes ausgehend, bis zu dem ersten beweglichen Werkzeugteil, mit dem es treibend verbunden ist.

Das Schneidwerkzeug umfasst mindestens ein beweglich gehaltenes Messer, welches das zweite Werkzeugteil bildet. Das Messer ist vorzugsweise ein Schiebemesser, das zur Durchtrennung von Gewebe in distaler Richtung bewegt wird. Dazu ist der zweite Abtrieb des Betätigungsgetriebes über ein Übertragungsmittel mit dem Messer, d.h. mit dem zweiten Werkzeugteil verbunden. Das Übertragungsmittel erstreckt sich von dem in der Nähe des proximalen Endes des Schaftes angeordneten zweiten Abtrieb des Betätigungsgetriebes durch den Schaft bis zu dem distalen Ende desselben und schließt dort an den zweiten Werkzeugteil an. Das Übertragungsmittel ist vorzugsweise ein Schubmittel, das eine distale gerichtete Antriebsbewegung des zweiten Abtriebs überträgt. Das Schubmittel kann eine Stange, ein Rohr oder ein anderes biegesteifes, Schubkräfte übertragendes Mittel sein. Alternativ kann als Schubmittel auch ein biegeschlaffes Mittel, wie z.B. ein Kunststoff- oder Metallband, vorgesehen sein, das in einem engen Kanal seitlich so geführt ist, dass es nur axial beweglich ist, seitlich aber nicht ausweichen kann.

Vorzugsweise sind das Segmentzahnrad und die Kurvenscheibe miteinander drehfest verbunden, wobei das Segmentzahnrad mit der Zahnstange erst dann in Eingriff kommt, wenn die Kurvenscheibe einen Kurvenfolger zur Betätigung des ersten Werkzeugteils vollständig oder nahezu vollständig in eine Position überführt hat, in der das Zangenwerkzeug geschlossen ist. In diesem Sinne bilden die Zahnstange und das Segmentzahnrad eine formschlüssige Kupplungsanordnung, die eine Antriebsverbindung zwischen der Handbetätigungseinrichtung und dem zweiten Abtrieb an einer vorgegebenen Stelle des Bedienwegs der Handbetätigungseinrichtung herstellt und trennt, d.h. ein- und auskuppelt.

Die Kurvenscheibe weist mindestens einen ansteigenden Abschnitt mit winkelabhängig zunehmendem Radius und einen zweiten Abschnitt auf, an dem der Radius der Kurvenscheibe unabhängig vom Drehwinkel konstant ist oder bei Weiterdrehung in Betätigungsrichtung sogar wieder leicht abnimmt. Der Abschnitt mit winkelabhängig zunehmendem Radius kann ein Spiralbogen sein. Der Abschnitt, an dem der Radius der Kurvenscheibe unabhängig vom Drehwinkel konstant ist, kann ein zu der Drehachse konzentrischer Kreisbogen sein. Durch die winkelmäßige Abstimmung des Übergangs zwischen ansteigendem und nichtansteigendem Abschnitt auf das Segmentzahnrad, bzw. die von ihm festgelegte Kupplungsstelle, kann eine gewünschte Koordination der beiden Bewegungen der Werkzeugteile zueinander erreicht werden. Z.B. können die Bewegungen der beiden Werkzeugteile nacheinander mit einer dazwischen liegenden Pause, ein anschließender Übergang ohne Pause oder ein überlappender Übergang festgelegt werden.

Bevorzugterweise ist die Kupplungsstelle, bei der die Verzahnung des Segmentzahnrads mit der Zahnstange in Eingriff kommt, in der Drehposition angeordnet, bei der der ansteigende Abschnitt der Kurvenscheibe in den nichtansteigenden Abschnitt übergeht. Außerdem sind weitere konstruktive Freiheitsgrade vorhanden, die unabhängig voneinander genutzt werden können, um die Längen der Wegabschnitte des Betätigungswegs der Handbetätigungseinrichtung auf die gewünschten Bewegungen der Werkzeugteile abzustimmen. Ein solcher Parameter ist beispielsweise der Durchmesser des Segmentzahnrads sowie der Anstieg des Radius an der Kurvenscheibe.

Insgesamt wird eine einfache Kinematik erhalten, die zudem mit geringen Betätigungskräften auskommt. Der Übergang zwischen der Bewegung der beiden Werkzeugteile kann gleitend und ohne damit verbundenes Knackgeräusch oder dergleichen dem Chirurgen irritierendes Verhalten bewerkstelligt werden. Außerdem ist es möglich, die Handbetätigungseinrichtung als Schwenkhebel auszuführen, der ein festes unveränderliches Schwenkzentrum aufweist. Das Drehzentrum des Schwenkhebels ist für das Schließen des Zangenwerkzeugs und für das Betätigen des Schneidwerkzeugs das gleiche. Ferner stellen sich für Fertigungstoleranzen bei dem erfindungsgemäßen Betätigungsgetriebe nur geringe Anforderungen, was die Produktionssicherheit und die Betätigungsqualität erhöht.

Der Antrieb des Betätigungsgetriebes erfolgt vorzugsweise über ein Übersetzungsgetriebe, das einen geringen Schwenkwinkel des zur Handbetätigung eingerichteten Schwenkhebels in eine Drehung der Kurvenscheibe und des Bogenzahnrads um einen größeren Drehwinkel bewirkt. Ein solches Übersetzungsgetriebe kann ein Zugmittelgetriebe sein, bei dem ein Zugmittel mit einem Ende an einen Handhebel angeschlossen und mit seinem anderen Ende auf ein Wickelorgan, z.B. eine Seilrolle, aufgewickelt ist, von der es bei Betätigung des Handhebels abgezogen wird. Insbesondere aber kann das Übersetzungsgetriebe durch ein Bogenzahnrad und ein mit diesem kämmendes Zahnrad oder Ritzel gebildet sein, wobei das Bogenzahnrad mit dem Schwenkhebel verbunden ist. Das Zahnrad treibt das Segmentzahnrad und die Kurvenscheibe an.

Anstelle des Segmentzahnrads kann eine bogenförmige Zahnstange zum Einsatz kommen, die mit dem Handhebel z.B. starr verbunden ist. Weiter kann die Zahnstange auch gerade ausgebildet und an einem Ende schwenkbar mit dem Handhebel verbunden sein. Ist die Handbetätigungseinrichtung anstelle schwenkbeweglich linear beweglich an oder in dem Gehäuse gelagert, kann die gerade Zahnstange auch starr mit der Handbedieneinrichtung verbunden sein. Im einfachsten Falle sind die Kurvenscheibe, das Segmentzahnrad und das zum Antrieb dienende Zahnrad einstückig miteinander verbunden. Sie können beispielsweise als Kunststoff-Spritzgussteil oder auf anderem Wege als ein nahtloses Teil aus einheitlichem Material ausgebildet sein.

Weitere Einzelheiten vorteilhafter Ausführungsformen der Erfindung ergeben sich aus der Zeichnung, der zugehörigen Beschreibung oder Ansprüchen. Es zeigen:
Figur 1 das Instrument, in schematisierter Seitenansicht,
Figur 2 das Betätigungsgetriebe in teilweise stark schematisierter Prinzip-Darstellung,
Figur 3 das Betätigungsgetriebe nach Figur 2, in schematisierter Draufsicht,
Figur 4 ein Diagramm zur Veranschaulichung des Betriebs des Betätigungsgetriebes nach Figur 2 und 3 und
Figur 5 ein Diagramm zur Veranschaulichung einer abgewandelten Ausführungsform des Betätigungsgetriebes.

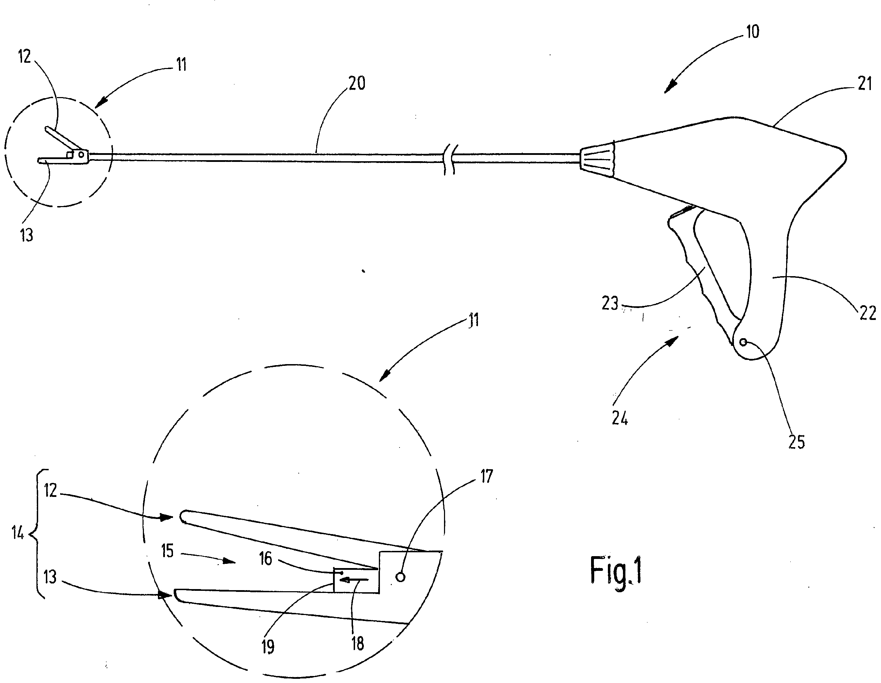

In Figur 1 ist ein chirurgisches Instrument 10 veranschaulicht, dass so wie es dargestellt ist, für laparoskopische Anwendungen vorgesehen ist. Das Werkzeug 11 ist zur Einwirkung auf biologisches Material, insbesondere zur Einwirkung auf Hohlgefäße eingerichtet, um diese verschließen und gegebenenfalls durchtrennen zu können. Wie die in Figur 1 unten angegebene Teildarstellung erkennen lässt, weist das Werkzeug 11 ein zwei Branchen 12, 13 umfassendes Zangenwerkzeug 14 sowie ein Schneidwerkzeug 15 auf, das durch ein linear beweglich gelagertes Messer 16 gebildet ist.

Von den beiden Branchen 12, 13 ist mindestens eine, in Figur 1 die Branche 12, um einen Scharnierbolzen 17 schwenkbar gelagert und bildet somit einen ersten beweglichen Werkzeugteil des Werkzeugs 11. Das Messer 16 der Schneideinrichtung 15 bildet einen zweiten beweglichen Werkzeugteil. Im vorliegenden Ausführungsbeispiel ist das Messer 16 bei Aktivierung in einer durch einen Pfeil 18 angedeuteten Richtung zur Durchtrennung von Gewebe in dista-ler Richtung beweglich. Das Messer 16 läuft dabei in einer in der unteren Branche 14 vorgesehenen Vertiefung oder Nut. Ebenso kann in der oberen Branche 12 eine entsprechende Vertiefung oder Nut vorgesehen sein, um das Messer 16 bei geschlossenem Zangenwerkzeug 14 bewegen zu können. Dabei durchdringt eine vordere Schneidkante 19 zwischen den Branchen 12, 13 gefasstes Gewebe.

An den Branchen 12, 13 können nicht weiter veranschaulichte Mittel zur Einwirkung auf biologisches Gewebe, wie beispielsweise Elektroden, Vorsprünge, Zähne, Vertiefungen, Kanten oder dergleichen, vorgesehen sein, beispielsweise um zwischen den Branchen 12, 13 gefasstes Gewebe zu koagulieren oder Gefäße zu fusionieren. Das Messer 16 kann ein mechanisches Messer mit scharfer Schneidkante 19 oder auch ein elektrisch schneidendes oder elektrisch unterstützt schneidendes Messer sein, das zum Durchtrennen von Gewebe sowohl mechanisch bewegbar und/oder mit einer zum Unterstützen oder Bewirken des Schneidvorgangs geeigneten elektrischen Spannung beaufschlagbar ist.

Das Werkzeug 11 ist an dem distalen Ende eines länglichen Schafts 20 angebracht, dessen proximales Ende mit einem Gehäuse 21 verbunden ist. Letzteres weist einen Handgriff 22 auf, zu dem auch ein Handhebel 23 gehört. Dieser bildet eine Handbetätigungseinrichtung 24. Der Handhebel 23 ist an einer geeigneten Stelle des Gehäuses 21, beispielsweise an einem unteren Ende des Handgriffs 22 an einer entsprechenden Lagerstelle 25 beweglich, vorzugsweise schwenkbar gelagert.

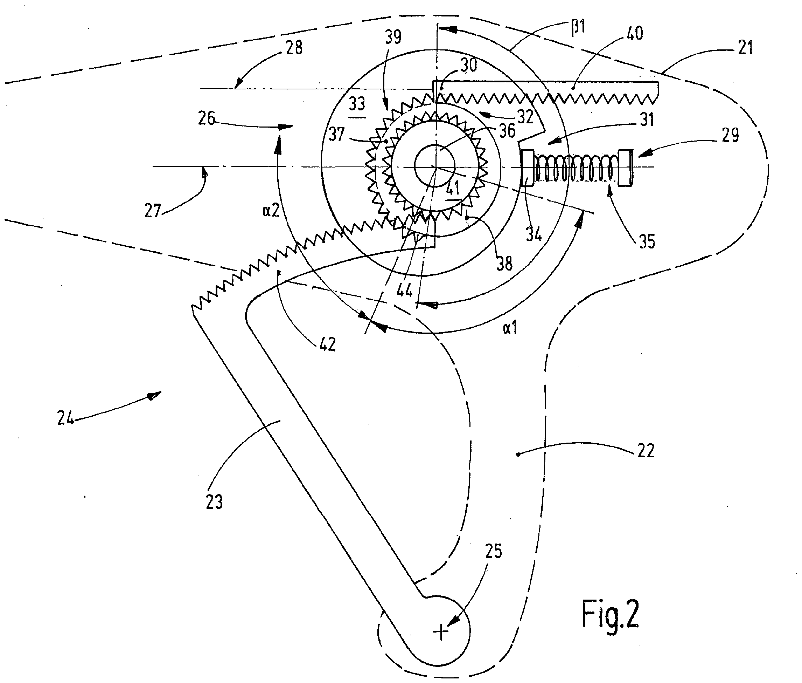

Das Gehäuse 21 umschließt ein in Figur 2 veranschaulichtes Betätigungsgetriebe 26 zur koordinierten Betätigung der beweglichen Werkzeugteile 12, 16 des Werkzeugs 11. Das Betätigungsgetriebe 26 setzt dabei eine Schwenkbewegung des Handhebels 23 in eine koordinierte Zug/Schub-Bewegung eines Zugmittels 27 und eines Schubmittels 28 um, die an ihrem jeweiligen distalen Ende mit den beweglichen Werkzeugteilen 12, 16 und an ihrem jeweiligen proximalen Ende mit einem ersten Abtrieb 29 bzw. dem zweiten Abtrieb 30 des Betätigungsgetriebes verbunden sind. Das erste Betätigungsmittel 27 ist ein Zugmittel, wie beispielsweise ein Draht, ein Seil, ein Band oder dergleichen aus Metall oder aus einem Kunststoff. Es kann biegeschlaff oder alternativ auch biegesteif ausgebildet sein. Jedenfalls ist es zugfest zur im Wesentlichen starren Übertragung einer Zugbewegung von dem ersten Abtrieb 29 auf das erste Werkzeugteil 12 eingerichtet. Bedarfsweise kann das Zugmittel 27 auch in Längsrichtung etwa federnd ausgebildet sein.

Das Schubmittel 28 dient zur Betätigung des Schneidwerkzeugs 15 und somit zum distalen Verschieben des Messers 16. Während das distale Ende des Schubmittels 18 mit dem Messer 16 verbunden ist, ist sein proximales Ende an den zweiten Abtrieb 30 des Betätigungsgetriebes 26 angeschlossen. Das Schubmittel 28 ist vorzugsweise schubsteif und dazu beispielsweise als Rohr oder als Profilstange mit Rundprofil, Rechteckprofil als Voll- oder Hohlprofil, U-Profil oder dergleichen ausgebildet. Als Schubmittel 28 kann auch ein biegeschlaffes Element, wie beispielsweise ein Kunststoff- oder Stahlband dienen, das in einem entsprechenden Kanal geführt ist. Alternativ kann das Schubmittel 28 ein wenig biegesteifes Kunststoff- oder Metallprofil, beispielsweise ein U-Profil, sein, dessen seitliches Ausknicken verhindert wird, indem es z.B. auf einer Rippe in Längsrichtung durch den Schaft geführt ist.

Das Betätigungsgetriebe 26 umfasst ein Kurvengetriebe 31, das den ersten Abtrieb 29 aufweist, sowie ein Zahnsegmentgetriebe 32, zu dem der zweite Abtrieb 30 gehört.

Zu dem Kurvengetriebe 31 gehören eine Kurvenscheibe 33 und ein Kurvenfolgerglied 34, beispielsweise in Gestalt eines Gleitelements, einer Rolle oder dergleichen, das oder die an dem Umfang der Kurvenscheibe 33 entlang läuft. Das Kurvenfolgerglied 34 ist dazu linear in Längsrichtung des Zugmittels 27 und radial zu der Kurvenscheibe 33 beweglich gelagert und durch ein zwischen dem ersten Abtrieb 29 und dem Kurvenfolgerglied 34 wirksames Federmittel 35, zum Bei⁻. spiel in Gestalt einer Schraubenfeder, an den Umfang der Kurvenscheibe 33 angedrückt. Anstelle des Federmittels 35 kann zwischen dem Kurvenfolgerglied 34 und dem ersten Abtrieb 29 auch eine starre Verbindung vorgesehen sein, wenn das Zugmittel 27 in Längsrichtung federnd ausgebildet ist und insoweit als Zugfeder wirkt. in anderen Fällen kann auf ein Federmittel auch ganz verzichtet werden.

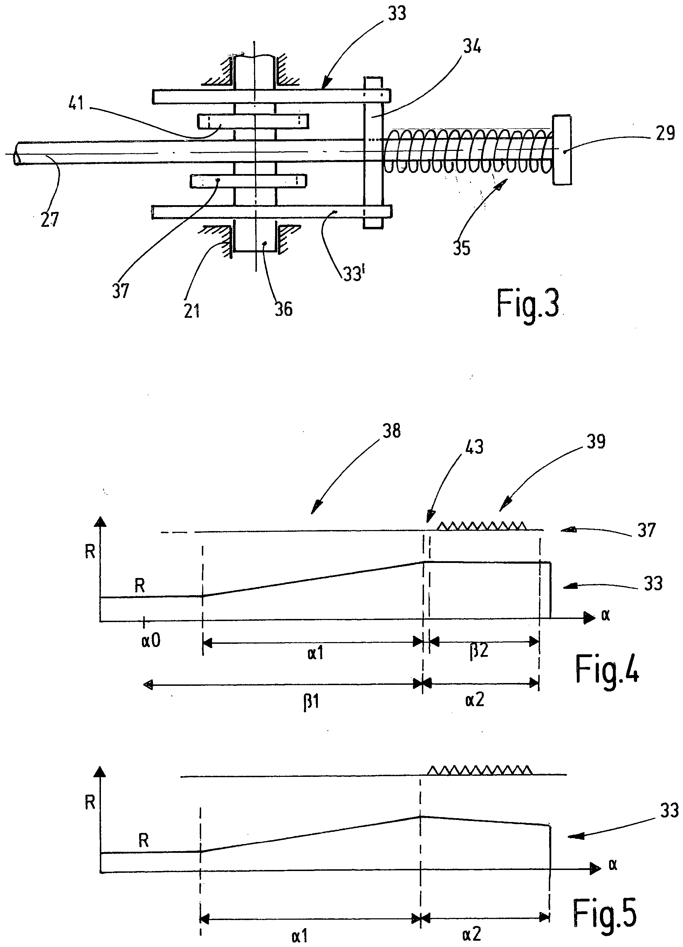

Der Umfang der Kurvenscheibe 33 weist einen ersten Winkelabschnitt al auf, in dem der Radius bei einer in Betätigungsrichtung erfolgenden Drehung der Kurvenscheibe 33 (in Figur 2 Drehung gegen den Uhrzeigersinn) zunimmt. An den Winkelabschnitt α1 schließt sich ein Winkelbereich α2 an, in dem der Radius der Kurvenscheibe 33 winkelunabhängig ist. Figur 4 veranschaulicht die Zunahme des Radius R in dem Winkelabschnitt α1 und die Konstanz des Radius R in dem Winkelabschnitt α2 in einem kartesischen Diagramm, das als Abwicklung der Kurvenscheibe 33 verstanden werden kann.

Die Kurvenscheibe 33 ist auf einer aus Figur 3 ersichtlichen Welle 36 in dem Gehäuse 21 drehbar gelagert, wobei diese Welle 36 eine zweite deckungsgleiche Kurvenscheibe 33' tragen kann, die ebenfalls mit dem Kurvenfolgerglied 34 in Berührung steht, damit dieses von den beiden synchron drehenden Kurvenscheiben 33, 33' linear bewegt werden kann. Das Zugmittel 27 ist in Figur 2 lediglich anhand einer strichpunktierten Linie in seiner Wirkrichtung dargestellt. Es kann eine Kröpfung, eine Ausnehmung oder dergleichen aufweisen, um, wie aus Figur 3 ersichtlich, um die Welle 36 herum geführt zu werden.

Mit der oder den Kurvenscheiben 33, 33a drehfest verbunden ist ein zu dem Zahnsegmentgetriebe 32 gehöriges Segmentzahnrad 37, das einen zahnlosen Abschnitt 38 und einen mit Zähnen versehenen Abschnitt 39 aufweist. Das Segmentzahnrad 37 ist in Figur 4 oberhalb der Abwicklung der Kurvenscheibe 33 ebenfalls als Abwicklung eingetragen. Wie ersichtlich, sind die Abschnitte 38, 39 zumindest bei einer bevorzugten Ausführungsform so angeordnet, dass der zahnlose Abschnitt 38 und der erste Winkelabschnitt α1 der Kurvenscheibe 33 wirkungsmäßig überlappen. Zur Erläuterung wird nochmals auf Figur 2 Bezug genommen, die eine zu dem Zahnsegmentgetriebe 32 gehörige, in dem Gehäuse 21 linear in Richtung des Schubmittels 28 beweglich gelagerte Zahnstange 40 veranschaulicht. Die Zahnstange 40 kann an einem Schlitten gelagert sein, der zugleich den zweiten Abtrieb 30 bildet oder sie selbst kann als solcher Abtrieb dienen. Der zahnlose Bereich 38 des Segmentzahnrads 37 nimmt einen Winkelbereich β1 ein, der keine Antriebsverbindung zwischen dem Segmentzahnrad 37 und der Zahnstange 40 zulässt. Während der zahnlose Abschnitt 38 in Figur 4 mit dem ansteigenden Winkelabschnitt α1 der Kurvenscheibe 33 in Übereinstimmung dargestellt ist, ist in Figur 2 ein realer Winkelversatz zwischen diesen Bereichen zu verzeichnen. Dies, weil bei der realen Ausführung gemäß Figur 2 das Kurvenfolgerglied 34 und der Zahnstangenanfang der Zahnstange 40 aus Sicht der Welle 36 winkelmäßig gegeneinander versetzt sind, so dass das Zahnsegmentgetriebe 32 und das Kurvengetriebe 31 unterschiedliche Nullpunkte haben. Diese Nullpunkte sind in Figur 4 in Übereinstimmung gebracht. Damit ist auch erkennbar, dass der mit Zähnen versehene Abschnitt 39 des Segmentzahnrads 37 in einem Winkelbereich β2 aktiv ist, d.h. mit der Zahnstange 40 koppelt, in dem das Kurvenfolgerglied 34 über den zweiten Winkelbereich α2 der Kurvenscheibe 33 läuft.

Zum drehenden Antrieb der Kurvenscheibe 33 (und 33') sowie des Segmentzahnrads 37 dient ein weiteres Zahnrad 41, das mit einem Zahnsegment 42 koppelt. Das Zahnsegment 42 ist mit dem Handhebel 23 verbunden, der zum Beispiel gegen die Kraft einer nicht weiter veranschaulichten Feder zu dem Handgriff 22 hin geschwenkt werden kann. Dabei dreht das Zahnsegment 42 das Zahnrad 41 und mit diesem sowohl die Kurvenscheibe 33 als auch das Segmentzahnrad 37.

Das insoweit beschriebene Instrument 10 arbeitet wie folgt:
Zu Beginn des Einsatzes befindet sich das Instrument 10 in der in Figur 1 veranschaulichten Position. Das Zangenwerkzeug 14 ist offen und das Schneidwerkzeug 15 inaktiv, d.h. das Messer 16 in proximal zurückgezogener Position. Der Handhebel 23 befindet sich in einer Ausgangsposition, ähnlich Figur 1 und 2, in der ein Ende des Zahnsegments 42 mit dem Zahnrad 41 in Eingriff steht. Das Segmentzahnrad 37 ist außer Eingriff mit der Zahnstange 40 und das Kurvenfolgerglied 34 befindet sich in einer Position der Kurvenscheibe 33, bei der die Kurvenscheibe 33 einen geringen Radius R aufweist. In Figur 4 entspricht dies einer Drehposition α0 für die Welle 36 und der einstückig mit ihr verbundenen Kurvenscheiben 33, 33' und Zahnräder 37, 41.

Wird der Handhebel 23 nun zu dem Griff 22 hin geschwenkt, dreht die Welle 36, womit das Kurvenfolgerglied 34 entlang des ersten Winkelabschnitts α1 der Kurvenscheibe 33 gleitet und dadurch in Proximalrichtung läuft. Diese Bewegung wird über das Federmittel 35 oder eine sonstige Verbindung auf den ersten Abtrieb 29 übertragen, so dass das Zugmittel 27 in proximaler Richtung bewegt und somit das Zangenwerkzeug 14 geschlossen wird. Die vollständige Schließung ist vorzugsweise erreicht, bevor die Kurvenscheibe 33 die Drehung innerhalb des ersten Winkelabschnitts α1 vollständig absolviert hat, so dass das Federmittel 35 am Übergang 43 zwischen dem Winkelabschnitt α1 und dem Winkelbereich α2 gespannt (komprimiert) ist.

In dem Übergangsabschnitt 43 kann die Krümmung von der Spiralform des Abschnitts α1 allmählich auf die zu der Welle 36 konzentrische Kreisbogenform des Winkelbereichs α2 übergehen. In oder nach dem Übergang kuppeln das Segmentzahnrad 37 und die Zahnstange 40 ein. Dabei gelangt der erste Zahn 44 des Abschnitts 39 in Zahneingriff mit der Zahnstange 40 so dass eine weitere Drehung der Welle 36 nun eine distale Verschiebung der Zahnstange 40 bewegt. Diese distale Verschiebung wird über das Schubmittel 28 auf das Messer 16, d.h. auf das Schneidwerkzeug 15 übertragen, so dass das zwischen beiden Branchen 12, 13 festgehaltene biologische Material, beispielsweise ein Gefäß durchtrennt wird.

Es versteht sich, dass geeignete Hemm- oder Sperrmittel vorgesehen sein können, um eine Drehung der Welle 36 bei Erreichen des Übergangsabschnitts 43 vorerst zu sperren, um beispielsweise bei geschlossenem Zangenwerkzeug 14 eine Koagulation des Gefäßes oder sonstigen Materials zwischen den Branchen 12 und 13 bewirken zu können. Entsprechende Sperrmittel zur vorläufigen Sperrung der weiteren Bewegung, Aktivierungsmittel für Koagulations- und andere Elektroden, Schalter und dergleichen sind in Figur 2 nicht veranschaulicht, können aber je nach Bedarf angebracht sein.

Figur 5 veranschaulicht eine leicht abgewandelte Ausführungsform des vorstehend beschriebenen Betätigungsgetriebes 26 anhand eines Diagramms. Der Unterschied liegt in der Ausgestaltung der Kurvenscheibe 33 sowie entsprechend 33' in dem zweiten Winkelbereich a2. Während der Radius R bei der Ausführungsform nach Figur 4 in diesem Abschnitt konstant ist, kann er gemäß Figur 5 in Drehrichtung a auch etwas abnehmen. Der Betrag der Steigung des Bereichs a2 ist aber vorzugsweise geringer als der Betrag der Steigung in dem Winkelabschnitt a1. Insbesondere ist die Radiusabnahme in dem Winkelbereich a2 so gering, dass das Federmittel 35 jedenfalls gespannt und somit Zangenwerkzeug 14 geschlossen bleibt, um das Gefäß während der Schneidphase festzuhalten. Das Gefälle in dem Winkelbereich a2 kann jedoch zur Verringerung der Betätigungskraft dienen, indem die Kurvenscheibe 33 in diesem Winkelbereich die Drehung der Welle 36 unterstützt.

Bei dem erfindungsgemäßen Instrument 10 ist ein Betätigungsgetriebe 26 vorhanden, das, ausgehend von der Betätigung einer Handbetätigungseinrichtung 24, zwei Funktionen eines Werkzeugs 11 erbringt. Dazu weist das Betätigungsgetriebe 26 ein Kurvengetriebe 31 sowie ein Zahnsegmentgetriebe 32 auf. Der Umfang der Kurvenscheibe 33 des Kurvengetriebes 31 ist in einem ersten Abschnitt a1 eine Spiralkurve zur Erzeugung eines Hubs für die Schließung des Zangenwerkzeugs 14, während der Umfang der Kurvenscheibe 33 in einem zweiten, sich daran anschließenden Winkelbereich α2 auf einem Kreis liegt. Der tangentiale Übergang der Umfangsfläche des ersten Winkelabschnitts α1 zu dem zweiten Winkelbereich α2 kann als Umschaltpunkt zwischen den Bewegungsabläufen gesehen werden. Sobald die Kurvenscheibe 33 mit dem kreisförmigen Abschnitt das Federmittel 35 spannt, erfolgt keine weitere Bewegung an dem Abtrieb des Kurvengetriebes 31. Dies wird erreicht, indem das Kurvenfolgerglied 34 und die Achse 36 der Kurvenscheibe 33 mit dem Zugmittel 27 auf einer Linie liegen, so dass von der Kurvenscheibe 33 kein Gegenmoment auf den Handhebel 23 mehr ausgeübt wird.

Vorzugsweise sind zwei deckungsgleiche Kurvenscheiben 33, 34 vorgesehen, die mit dem antreibenden Zahnrad 41 und dem Segmentzahnrad 37 sowie der Welle 36 ein einteiliges Kunststoffspritzgussteil bilden. Dies führt zu einer geringen Teilezahl. Die rechte und eine linke Kurvenscheibe 33, 33' dienen zur symmetrischen Kraftübertragung. Der Bauraum zwischen beiden Kurvenscheiben 33, 33' kann für einen Schlitten mit Zahnstange 40 zur Messerverschiebung genutzt werden. Durch diese Verschachtelung der Bauteile wird der gesamte benötigte Bauraum reduziert, was die Ausführung eines kompakten und ergonomischen Gehäuses 21 ermöglicht.

**Bezugszeichen:**

| | |
|---|---|
| 10 | Instrument |
| 11 | Werkzeug |
| 12, 13 | Branchen |
| 14 | Zangenwerkzeug |
| 15 | Schneidwerkzeug |
| 16 | Messer |
| 17 | Scharnierbolzen |
| 18 | Pfeil |
| 19 | Schneidkante |
| 20 | Schaft |
| 21 | Gehäuse |
| 22 | Handgriff |
| 23 | Handhebel |
| 24 | Handbetätigungseinrichtung |
| 25 | Lagerstelle |
| 26 | Betätigungsgetriebe |
| 27 | Zugmittel |
| 28 | Schubmittel |
| 29 | erster Abtrieb |
| 30 | zweiter Abtrieb |
| 31 | Kurvengetriebe |
| 32 | Zahnsegmentgetriebe |
| 33, 33' | Kurvenscheibe |
| 34 | Kurvenfolgerglied |
| 35 | Federmittel |
| α1 | erster Winkelabschnitt der Kurvenscheibe 33 |
| α2 | zweiter Winkelbereich |
| R | Radius der Kurvenscheibe 33 |
| 35 | Federmittel |
| 36 | Welle |
| 37 | Segmentzahnrad |
| 38, 39 | Abschnitte des Segmentzahnrads |
| 40 | Zahnstange |
| β1, β2 | Winkelbereiche des Segmentzahnrads 37 |
| 41 | Zahnrad |
| 42 | Zahnsegment |
| α0 | Ausgangsposition der Welle 36 |
| 43 | Übergangsabschnitt zwischen α1 und α2 |
| 44 | erster Zahn des Abschnitts 37 |
| α | Drehrichtung |

## Patentansprüche

1. Instrument (10) zur Durchführung chirurgischer Eingriffe an einem Patienten,
mit einem Werkzeug (11), das wenigstens zwei zeitlich versetzt zu bewegende Werkzeugteile (12, 16) aufweist,
mit einem Betätigungsgetriebe (26), das einen ersten Abtrieb (29) für ein erstes der Werkzeugteile (12) und einen zweiten Abtrieb (30) für ein zweites der Werkzeugteile (16) aufweist, wobei das Betätigungsgetriebe (26) ein Kurvengetriebe (31) aufweist, das mit dem ersten Abtrieb (29) verbunden ist, mit einem ersten Übertragungsmittel (27), das einerseits mit dem ersten Werkzeugteil (12) und andererseits mit dem ersten Abtrieb (29) verbunden ist, mit einem zweiten Übertragungsmittel (28), das einerseits mit dem zweiten Werkzeugteil (16) und andererseits mit dem zweiten Abtrieb (30) verbunden ist,
**dadurch gekennzeichnet,**
**dass** das Betätigungsgetriebe (26) ein Zahnsegmentgetriebe (32) aufweist, das mit dem zweiten Abtieb (30) verbunden ist, wobei das Zahnsegmentgetrieb (32) ein Segmentzahnrad (37) und eine Zahnstange (40) aufweist die mit dem zweiten Abtrieb (30) verbunden ist.

2. Instrument nach Anspruch 1, **dadurch gekennzeichnet, dass** das Betätigungsgetriebe (26) eingangsseitig mit einer Handbetätigungseinrichtung (24) verbunden ist.

3. Instrument nach einem der vorstehenden Ansprüche, **dadurch gekennzeichnet, dass** zu dem Werkzeug (11) ein Zangenwerkzeug (14) gehört und dass der erste Werk-zeugteil (12) eine bewegliche Branche (12) des Zangenwerkzeugs (14) ist.

4. Instrument nach einem der vorstehenden Ansprüche, **dadurch gekennzeichnet, dass** zu dem Werkzeug (11) ein Schneidwerkzeug (15) gehört und dass der zweite Werkzeugteil (16) ein Messer (16) ist.

5. Instrument nach einem der vorstehenden Ansprüche, **dadurch gekennzeichnet, dass** das erste Übertragungsmittel (27) ein Zugmittel und dass das zweite Übertragungsmittel (28) ein Schubmittel ist.

6. Instrument nach einem der vorstehenden Ansprüche, **dadurch gekennzeichnet, dass** zu dem Kurvengetriebe (31) mindestens eine Kurvenscheibe (33) und mindestens ein Kurvenfolger (34) gehören, der mit der Kurvenscheibe (33) in Anlage steht, und dass der Kurvenfolger (34) mit dem ersten Abtrieb (29) verbunden ist.

7. Instrument nach Anspruch 6, **dadurch gekennzeichnet, dass** die Kurvenscheibe (33) wenigstens einen Abschnitt (α2) mit winkelunabhängig konstantem Radius (R) und wenigstens einen Abschnitt (α1) mit winkelabhängigem Radius (R) aufweist.

8. Instrument nach einem der vorstehenden Ansprüche, **dadurch gekennzeichnet, dass** zwischen dem Kurvengetriebe (31) und dem ersten Abtrieb (29) ein Federmittel (35) angeordnet ist.

9. Instrument nach Anspruch 1, **dadurch gekennzeichnet, dass** das Segmentzahnrad (37) einen zahnlosen Umfangsabschnitt (38) und einen mit Zähnen versehenen Umfangsabschnitt (39) aufweist, die mit der Zahnstange (40) in kämmenden Eingriff überführbar sind.

10. Instrument nach Anspruch 6, **dadurch gekennzeichnet, dass** die Kurvenscheibe (33) und das Segmentzahnrad (37) drehfest miteinander verbunden sind.

11. Instrument nach Anspruch 10, **dadurch gekennzeichnet, dass** das Segmentzahnrad (37) und die Kurvenscheibe (33) in Bezug aufeinander derart angeordnet sind, dass das Segmentzahnrad (37) bei Betätigung des Werkzeugs (11) erst dann mit der Zahnstange (40) in Eingriff gelangt, wenn der ansteigende Abschnitt (α1) der Kurvenscheibe (33) den Kurvenfolger (34) passiert hat.

12. Instrument nach einem der vorstehenden Ansprüche **dadurch gekennzeichnet, dass** das Betätigungsgetriebe (26) ein Übersetzungsgetriebe zur Umsetzung einer Antriebbewegung einer Handbetätigungseinrichtung (24) in eine Drehung der Kurvenscheibe (33) und des Segmentzahnrads (37) aufweist.

13. Instrument nach einem der vorstehenden Ansprüche **dadurch gekennzeichnet, dass** das Betätigungsgetriebe (26) ein Zahnsegment (42) enthält, das mit einem Zahnrad (41) in kämmenden Eingriff steht, das mit der Kurvenscheibe (33) und mit dem Segmentzahnrad (37) treibend verbunden ist.

14. Instrument nach Anspruch 13, **dadurch gekennzeichnet, dass** das Zahnsegment (42) mit einem als Handbetätigungseinrichtung (24) dienenden Schwenkhebel (23) verbunden ist.

## Claims

1. Instrument (10) for performing surgical interventions on a patient,
with a tool (11) having at least two tool parts (12, 16) which are to be moved with a temporal offset,
with an actuating gear mechanism (26) having a first output (29) for a first of the tool parts (12) and a second output (30) for a second of the tool parts (16), wherein the actuating gear mechanism (26) has a cam gear mechanism (31) which is connected to the first output (29),
with a first transmission means (27) which is connected on the one side to the first tool part (12) and on the other side to the first output (29),
with a second transmission means (28) which is connected on the one side to the second tool part (16) and on the other side to the second output (30),
**characterised in that**
the actuating gear mechanism (26) has a toothed segment gear mechanism (32) which is connected to the second output (30), wherein the toothed segment gear mechanism (32) has a segment gear wheel (37) and a toothed rod (40) which is connected to the second output (30).

2. Instrument according to claim 1, **characterised in that** on the input side, the actuation gear mechanism (26) is connected to a manual actuation device (24).

3. Instrument according to one of the preceding claims, **characterised in that** a pincer tool (14) belongs to the tool (11), and the first tool part (17) is a movable branch (12) of the pincer tool (14).

4. Instrument according to any of the preceding claims, **characterised in that** a cutting tool (15) belongs to the tool (11), and the second tool part (16) is a cutter (16).

5. Instrument according to any of the preceding claims, **characterised in that** the first transmission means (27) is a tension means, and the second transmission means (28) is a thrust means.

6. Instrument according to any of the preceding claims, **characterised in that** at least one cam disc (33) and at least one cam follower (34), which is in contact with the cam disc (33), belong to the cam gear mechanism (31), and that the cam follower (34) is connected to the first output (29).

7. Instrument according to claim 6, **characterised in that** the cam disc (33) has at least one portion (α2) with constant angle-independent radius (R), and at least one portion (α1) with angle-dependent radius (R).

8. Instrument according to any of the preceding claims, **characterised in that** a spring means (35) is arranged between the cam gear mechanism (31) and the first output (29).

9. Instrument according to claim 1, **characterised in that** the segment gear wheel (37) has a toothless peripheral portion (38) and a peripheral portion (39) which is provided with teeth and can be transferred into meshing engagement with the toothed rod (40).

10. Instrument according to claim 6, **characterised in that** the cam disc (33) and the segment gear wheel (37) are rotationally fixedly connected together.

11. Instrument according to claim 10, **characterised in that** the segment gear wheel (37) and the cam disc (33) are arranged relative to each other such that, on actuation of the tool (11), the segment gear wheel (37) first comes into engagement with the toothed rod (40) when the rising portion (α1) of the cam disc (33) has passed the cam follower (34).

12. Instrument according to any of the preceding claims, **characterised in that** the actuation gear mechanism (26) has a translation gear for converting a drive movement of a manual actuation device (24) into a rotation of the cam disc (33) and segment gear wheel (37).

13. Instrument according to any of the preceding claims, **characterised in that** the actuation gear (26) contains a toothed segment (42) which is in meshing engagement with a gear wheel (41) which is connected in driving fashion to the cam disc (33) and the segment gear wheel (37).

14. Instrument according to claim 13, **characterised in that** the toothed segment (42) is connected to a pivot lever (23) serving as a manual actuation device (24).

## Revendications

1. Instrument (10) destiné à réaliser des interventions chirur-gicales sur un patient,
comprenant un outil (11) qui présente au moins deux parties d'outil (12, 16) devant être déplacées avec un décalage dans le temps,
comprenant un mécanisme d'actionnement (26) qui présente un premier organe de sortie (29) pour une première des parties d'outil (12), et un deuxième organe de sortie (30) pour une deuxième des parties d'outil (16), le mécanisme d'actionnement (26) présentant un mécanisme à came (31) qui est relié au premier organe de sortie (29),
comprenant un premier moyen de transmission (27) qui est relié d'une part à la première partie d'outil (12) et d'autre part au premier organe de sortie (29),
comprenant un deuxième moyen de transmission (28) qui est relié d'une part à la deuxième partie d'outil (16) et d'autre part au deuxième organe de sortie (30),
**caractérisé en ce que** le mécanisme d'actionnement (26) présente un mécanisme à segments dentés (32) qui est relié au deuxième organe de sortie (30), le mécanisme à segments dentés (32) présentant une roue à segment denté (37) et une crémaillère (40) qui est reliée au deuxième organe de sortie (30).

2. Instrument selon la revendication 1, **caractérisé en ce que** le mécanisme d'actionnement (26) est relié côté entrée à un dispositif d'actionnement manuel (24).

3. Instrument selon l'une des revendications précédentes, **caractérisé en ce qu'**un outil à pince (14) fait partie de l'outil (11), et **en ce que** la première partie d'outil (12) est un mors (12) mobile de l'outil à pince (14).

4. Instrument selon l'une des revendications précédentes, **caractérisé en ce qu'**un outil de coupe (15) fait partie de l'outil (11), et **en ce que** la deuxième partie d'outil (16) est une lame (16).

5. Instrument selon l'une des revendications précédentes, **caractérisé en ce que** le premier moyen de transmission (27) est un moyen de traction, et **en ce que** le deuxième moyen de transmission (28) est un moyen de poussée.

6. Instrument selon l'une des revendications précédentes, **caractérisé en ce que** le mécanisme à came (31) comprend au moins une came (33) et au moins un suiveur de came (34) qui est en appui contre la came (33), et **en ce que** le suiveur de came (34) est relié au premier organe de sortie (29).

7. Instrument selon la revendication 6, **caractérisé en ce que** la came (33) présente au moins une partie (α2) avec un rayon (R) constant, indépendamment de l'angle, et au moins une partie (α1) avec un rayon (R) dépendant de l'angle.

8. Instrument selon l'une des revendications précédentes, **caractérisé en ce qu'**un moyen formant ressort (35) est disposé entre le mécanisme à came (31) et le premier organe de sortie (29).

9. Instrument selon la revendication 1, **caractérisé en ce que** la roue à segment denté (37) présente une portion périphérique (38) dépourvue de dents et une portion périphérique (39) pourvue de dents qui peuvent être amenées en prise d'engrènement avec la crémaillère (40).

10. Instrument selon la revendication 6, **caractérisé en ce que** la came (33) et la roue à segment denté (37) sont reliées de façon solidaire en rotation l'une à l'autre.

11. Instrument selon la revendication 10, **caractérisé en ce que** la roue à segment denté (37) et la came (33) sont disposées l'une par rapport à l'autre de telle façon que lors de l'actionnement de l'outil (11), la roue à segment denté (37) n'entre en prise avec la crémaillère (40) que lorsque la partie ascendante (al) de la came (33) est passée sur le suiveur de came (34).

12. Instrument selon l'une des revendications précédentes, **caractérisé en ce que** le mécanisme d'actionnement (26) présente un engrenage de transmission destiné à transformer un mouvement d'entraînement d'un dispositif d'actionnement manuel (24) en une rotation de la came (33) et de la roue à segment denté (37).

13. Instrument selon l'une des revendications précédentes, **caractérisé en ce que** le mécanisme d'actionnement (26) comporte un segment denté (42) qui est en prise d'engrènement avec une roue dentée (41) qui est reliée en vue de l'entraînement à la came (33) et à la roue à segment denté (37).

14. Instrument selon la revendication 13, **caractérisé en ce que** le segment denté (42) est relié à un levier pivotant (23) servant de dispositif d'actionnement manuel (24).
